# EUROPEAN PATENT APPLICATION

(11) **EP 1 288 836 A2**
(43) Date of publication of application: **05.03.2003**
(21) Application number: 02018264.8
(22) Date of filing: 22.08.2002
(51) Int. Cl.: G06F 19/00

(54) **Examination follow-up information management apparatus for performing the simple and certain follow-up examinations**

(30) Priority: 24.08.2001 JP 2001255090
(71) Applicant: Olympus Optical Co., Ltd., Tokyo (JP)
(72) Inventor: Hiyama, Keiichi, Hino-shi, Tokyo (JP); Ohishi, Hiroko, Hachioji-shi, Tokyo (JP); Omoto, Masakazu, Hachioji-shi, Tokyo (JP); Watai, Makoto, Northport, New York 11768 (US); Shibata, Hiroyuki, Yokohama-shi, Kanagawa (JP)
(74) Representative: von Hellfeld, Axel, Dr. Dipl.-Phys.

(57) **Abstract**

A filing apparatus constituting an examination follow-up information management apparatus is disposed in a hospital and is connected to an electronic endoscope apparatus for capturing endoscopic images. This filing apparatus is allowed to manage endoscopic images and the reservation of re-examinations (follow-up examinations) by storing endoscopic images of the patient captured by the electronic endoscope apparatus in relation to the patient information. It thereby makes possible to manage follow-up information for the simple and certain performance of follow-up examinations.

## Description

### 1. Field of the Invention

The present invention relates to an examination follow-up information management apparatus for managing information for performing the medical follow-up examinations.

### 2. Description of the Related Art

In recent years, various examinations have come to be performed at medical facilities, and in particular, image examinations have become important for diagnosis or the like.

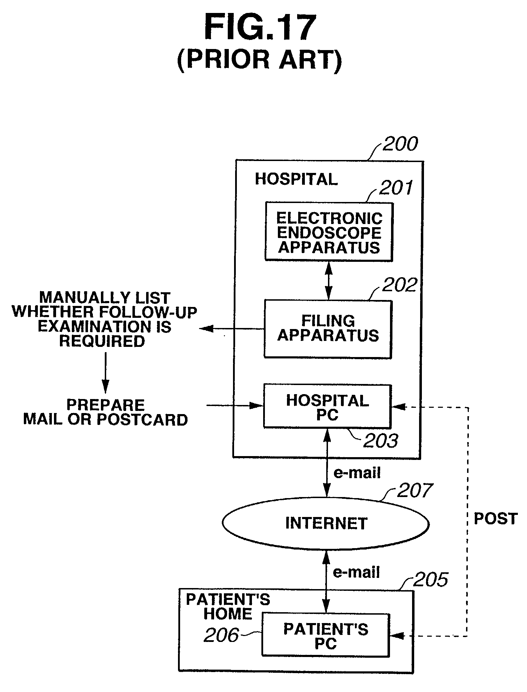

As shown in Figure 17, in these image examinations, medical image data are acquired with various types of medical examination instruments in a hospital 200, such as an electronic endoscope apparatus 201, and the medical image data attained are related to the patient information, and stored and managed in a filing apparatus 202.

The doctor determines the necessity for another examination (hereinafter "follow-up examination") by searching the abovementioned filing apparatus 202 after the examination and once more confirming the medical image data for each patient. When a follow-up examination is necessary, patients for whom follow-up examinations are required are listed; and then the chart or a hospital PC 203 is searched for the residential or e-mail address of the corresponding patient. E-mail is transmitted to a patient's PC 206 at a corresponding patient's home 205 through an Internet 207, or postal mail is sent to the patient's home 205, using e-mail software or address writing software in the hospital PC 203, whereby an appointment for the follow-up examination is made by the patient and the examination is performed.

However, a problem is that the doctor must list patients for whom examinations are necessary when conventionally scheduling the follow-up examinations, and time and effort are therefore required.

Another problem is that it is necessary to search charts or the hospital PC 203 for the listed patients' residential or e-mail addresses and a large amount of time is required to search (extract) information from charts in particular.

### OBJECTS AND SUMMARY OF THE INVENTION

It is an object of the present invention to provide an examination follow-up information management apparatus which is able to manage follow-up information for the simple and certain performance of follow-up examinations.

The examination follow-up information management apparatus relating to the present invention comprises: a display for displaying the examination image of the subject; a follow-up information input window display unit for displaying a follow-up information input window, for inputting follow-up information for the examination for the abovementioned subject, on the abovementioned display unit; a follow-up information input unit for inputting follow-up information for the abovementioned subject in the abovementioned follow-up information input window displayed on the abovementioned follow-up information input window display unit; and follow-up information memory for storing the abovementioned follow-up information input with the abovementioned follow-up information input unit as information for the abovementioned subject.

Other characteristics and advantages of the present invention will be made clear in the following explanation.

### BRIEF DESCRIPTION OF THE DRAWINGS

Figures 1 through 5 relate to a first embodiment of the present invention;
Figure 1 is a schematic diagram showing the constitution of the system of the filing apparatus comprising the examination follow-up information management apparatus;
Figure 2 is a flowchart for explaining the operation of the filing apparatus in Figure 1;
Figure 3 is a drawing showing a first image displayed in the process in Figure 2;
Figure 4 is a drawing showing a second image displayed in the process in Figure 2;
Figure 5 is a flowchart for explaining the process flow of the automatic distribution of the follow-up examination guidance and the reservation reception process in Figure 2;
Figures 6 through 14 relate to a second embodiment of the present invention;

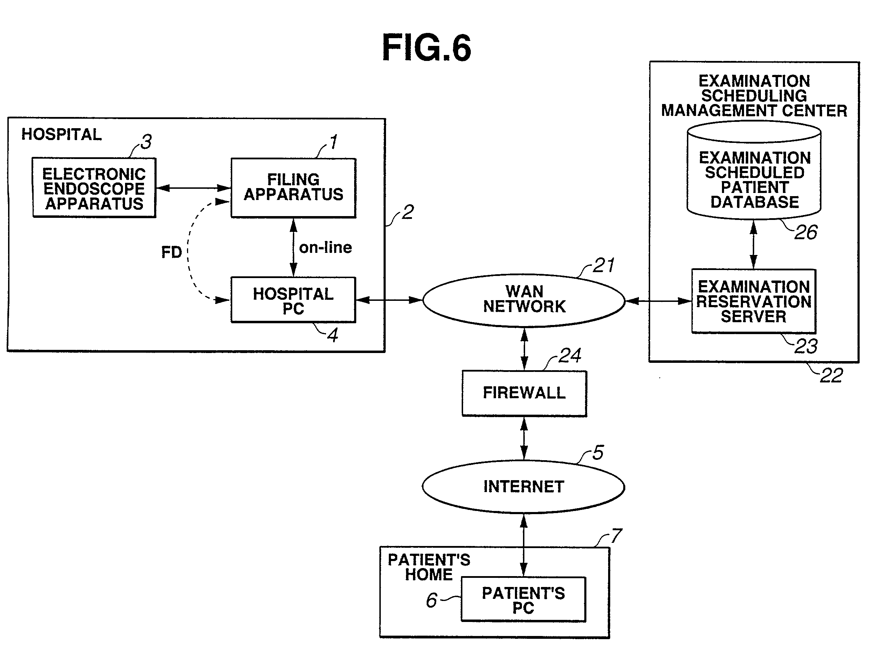

Figure 6 is a schematic diagram showing the constitution of a system comprising an examination reservation server 23 constituting the examination follow-up information management apparatus;

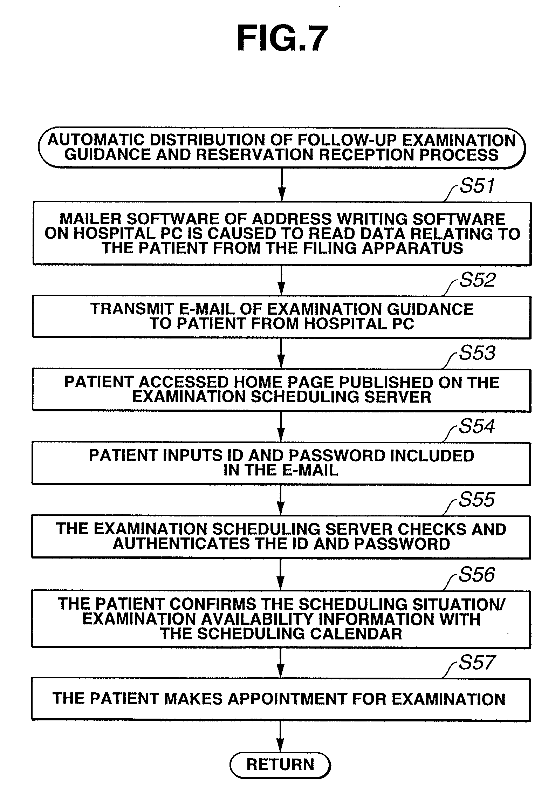

Figure 7 is a flowchart for explaining the process flow of the automatic distribution of the follow-up examination guidance and the reservation reception process in the examination reservation server in Figure 6;

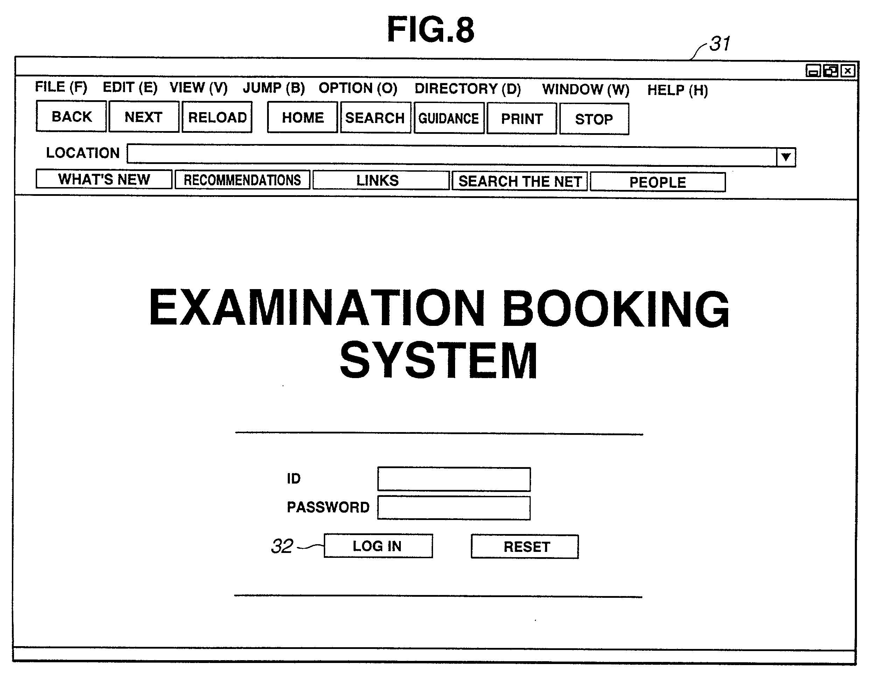

Figure 8 is a drawing showing the home page opened to the public in the process in Figure 7;

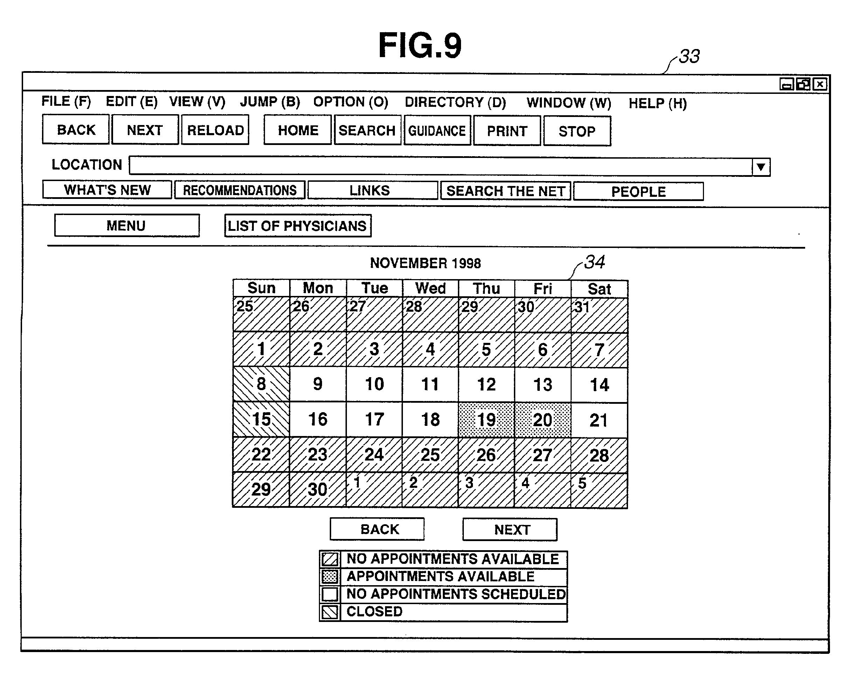

Figure 9 is a drawing showing the reservation scheduling calendar page opened to the public with the process in Figure 7;

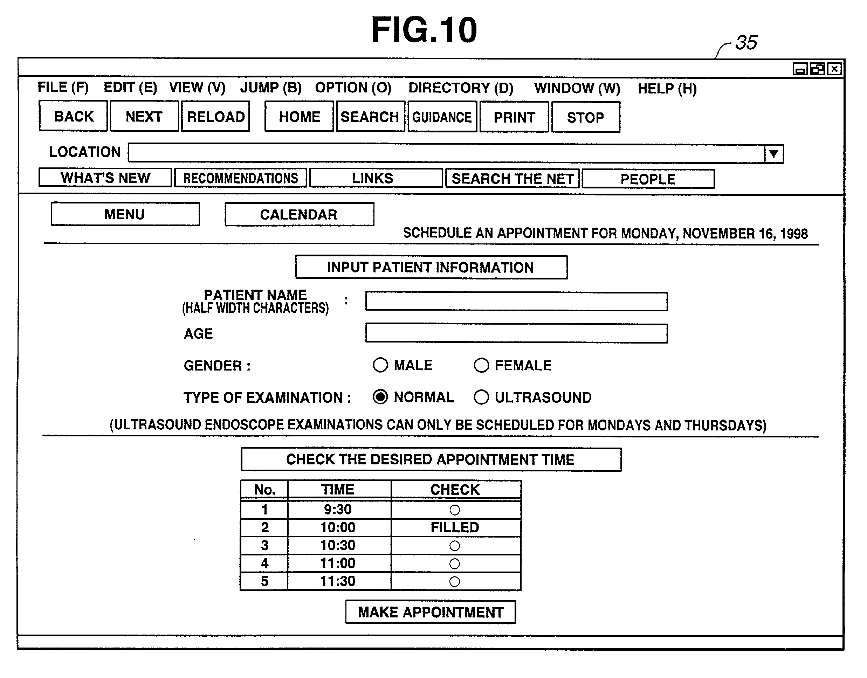

Figure 10 is a drawing showing the detailed reservation page opened to the public with the process in Figure 7;

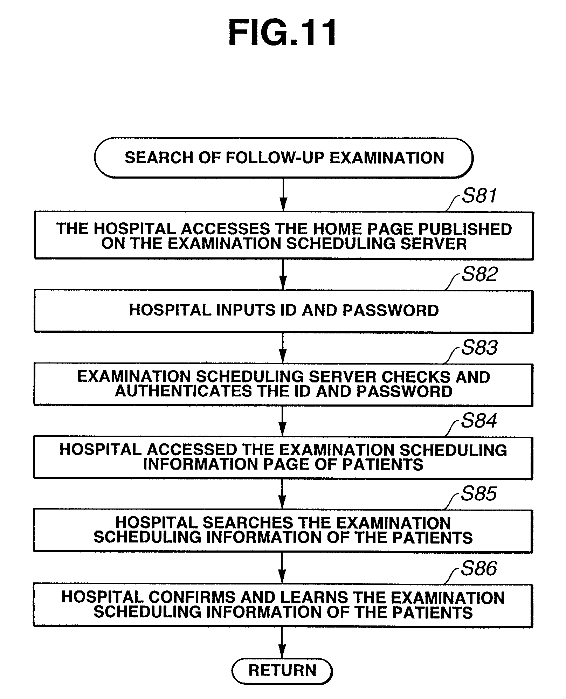

Figure 11 is a flowchart for explaining the flow of the process for reading the follow-up examination reservation situation of the patient with the examination reservation server in Figure 6;

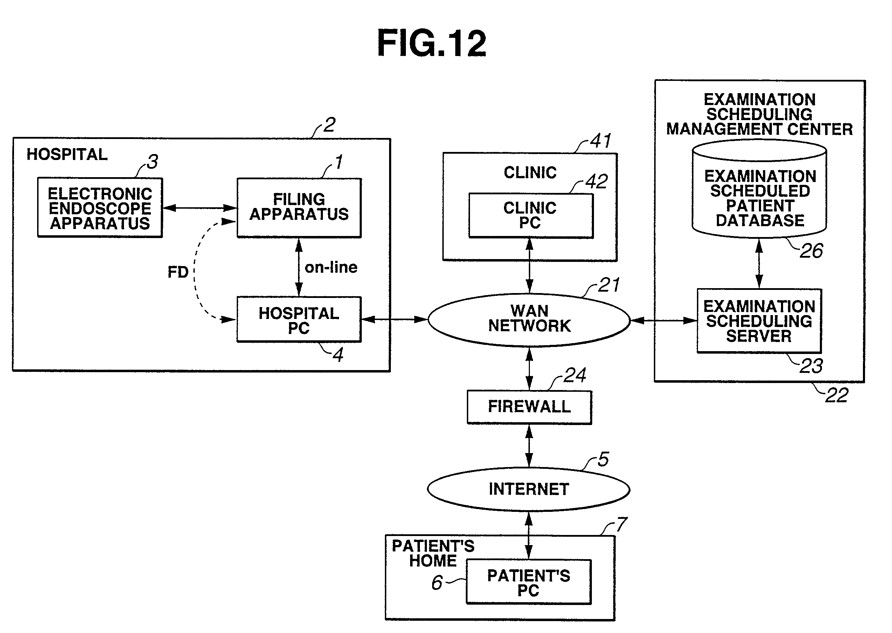

Figure 12 is a schematic diagram showing a first modification of the constitution of the system comprising the examination reservation server constituting the examination follow-up information management apparatus in Figure 6;

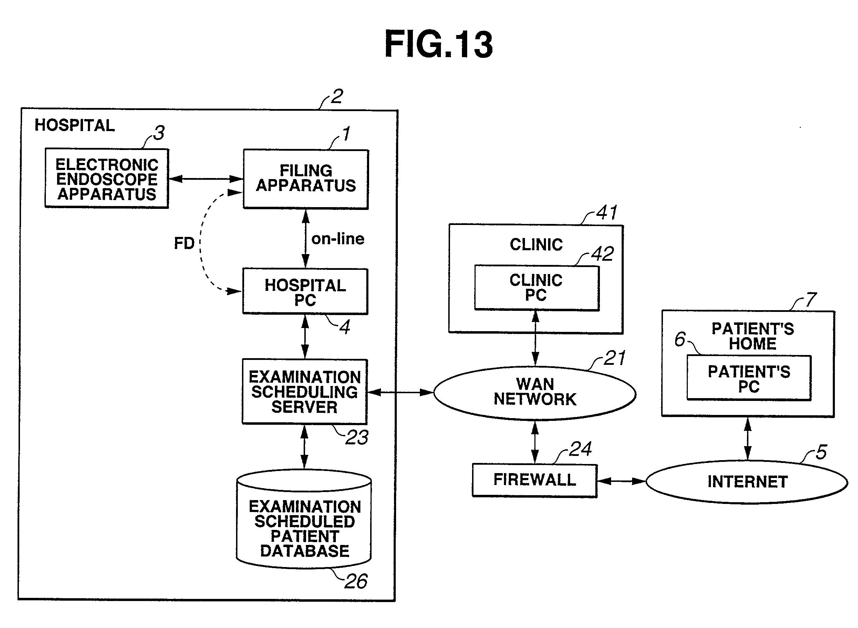

Figure 13 is a schematic diagram showing a second modification of the constitution of the system comprising the examination reservation server constituting the examination follow-up information management apparatus in Figure 6;

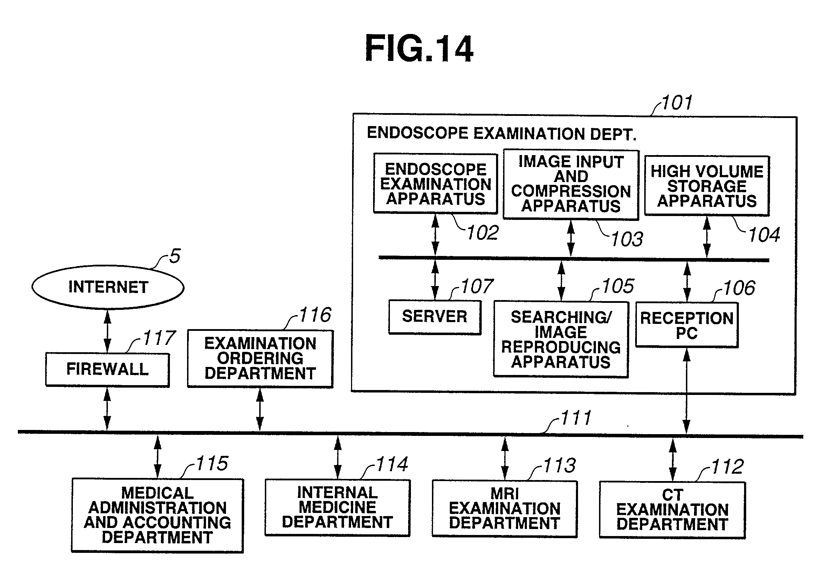

Figure 14 is a schematic diagram showing a third modification of the constitution of the system comprising the examination reservation server constituting the examination follow-up information management apparatus in Figure 6;

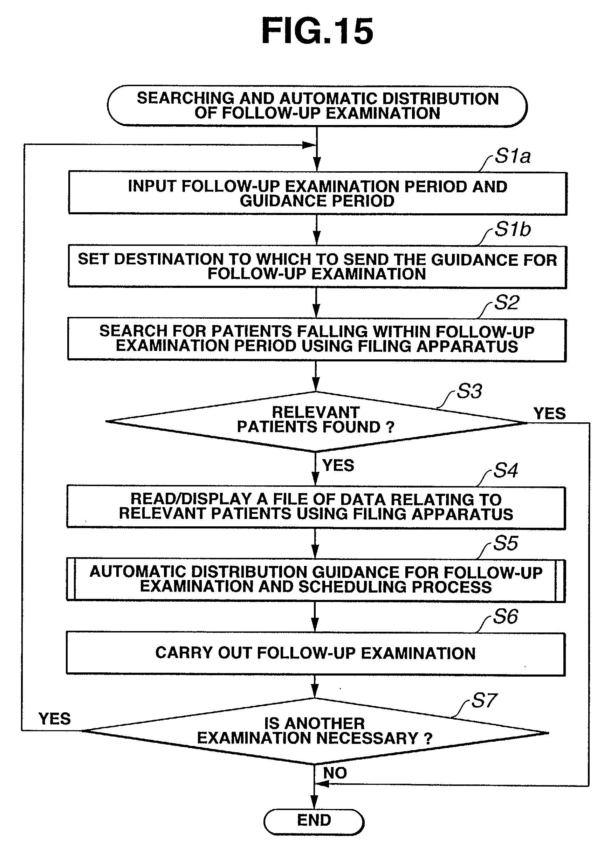

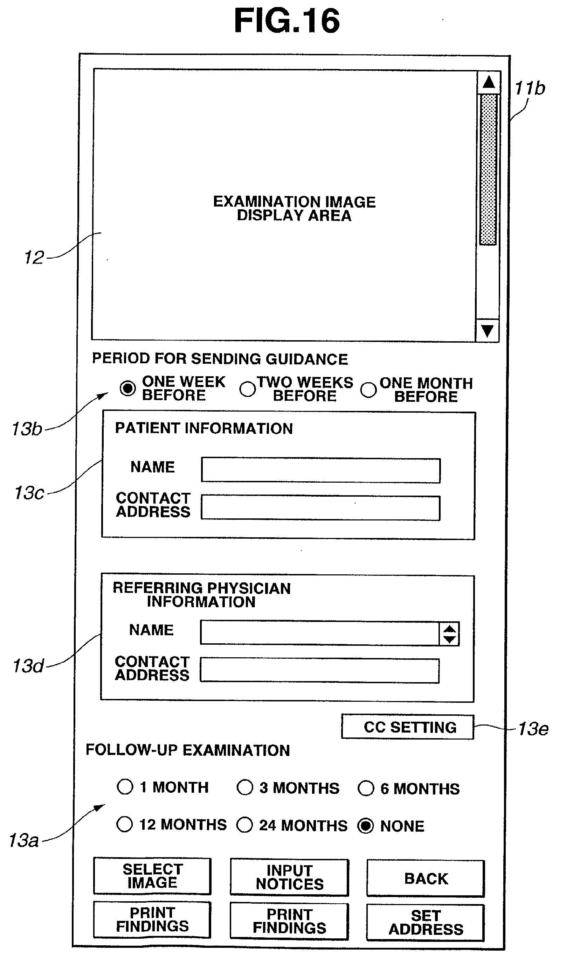

Figures 15 and 16 relate to a third embodiment of the present invention;
Figure 15 is a flowchart for explaining the operation of the filing apparatus;
Figure 16 is a drawing showing an image displayed in the process in Figure 15; and
Figure 17 is a schematic diagram showing the system constitution of the filing apparatus which made possible conventional reexamination.

### DESCRIPTION OF THE PREFERRED EMBODIMENTS

### First embodiment

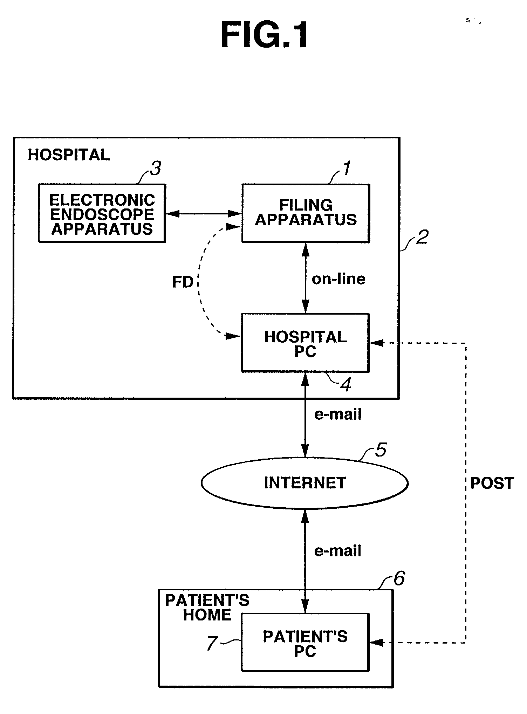

As shown in Figure 1, a filing apparatus 1 constituting the examination follow-up information management apparatus relating to the present embodiment is established in a hospital 2 and is connected to an electronic endoscope apparatus 3 for capturing endoscopic images.

Whereas, the filing apparatus 1 includes a display unit, a follow-up information input window display unit, a follow-up information input unit, a follow-up information memory, a follow-up information communication unit, a destination information memory, a follow-up information destination setting unit, a follow-up information destination setting window display unit, and a follow-up searching unit, etc.

This filing apparatus 1 is able to manage endoscopic images and the reexamination (follow-up examination) information by storing the endoscopic image of the patient captured with the electronic endoscope apparatus 3 in relation to the patient information.

Also, the filing apparatus 1 is connected to the Internet 5, or communication network, through a hospital personal computer (hereinafter "PC") 4. This hospital PC 4 plays the role of a firewall to prevent unauthorized access or the like to the filing apparatus 1 from the Internet 5.

The hospital PC 4 is connected to a patient's PC 7 in a patient's home 6 through the Internet 5. Electronic mail (hereinafter "e-mail") can be exchanged between the hospital PC 4 and the patient's PC 7.

The operation of the present embodiment constituted in this way is explained next.

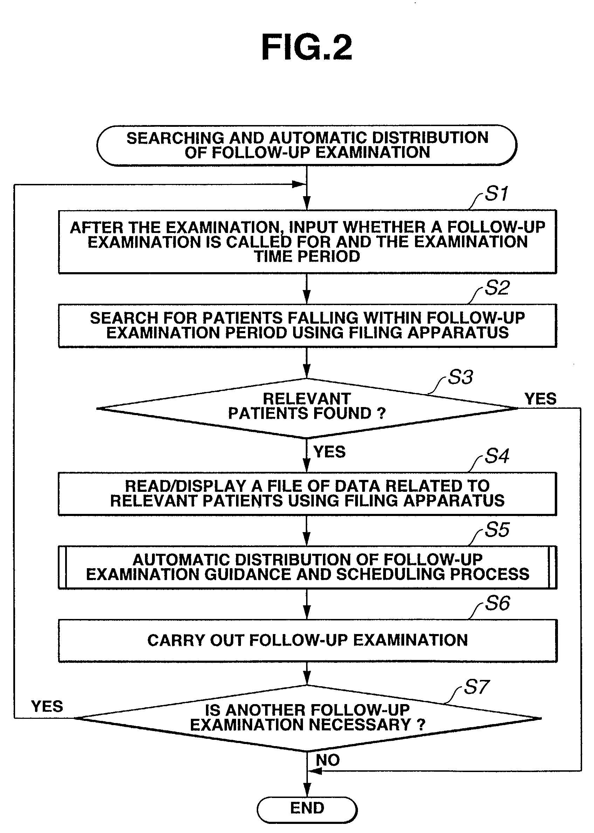

As shown in Figure 2, the endoscopic image is related to the patient information and stored in the filing apparatus 1 after the examination with the electronic endoscope apparatus 3. Thereupon, in Step S1, the doctor determines whether a follow-up examination (a reexamination after the passage of a prescribed period of time) with the endoscope is necessary for the patient, and then inputs whether a follow-up examination is to be given and the time of examination in the filing apparatus 1.

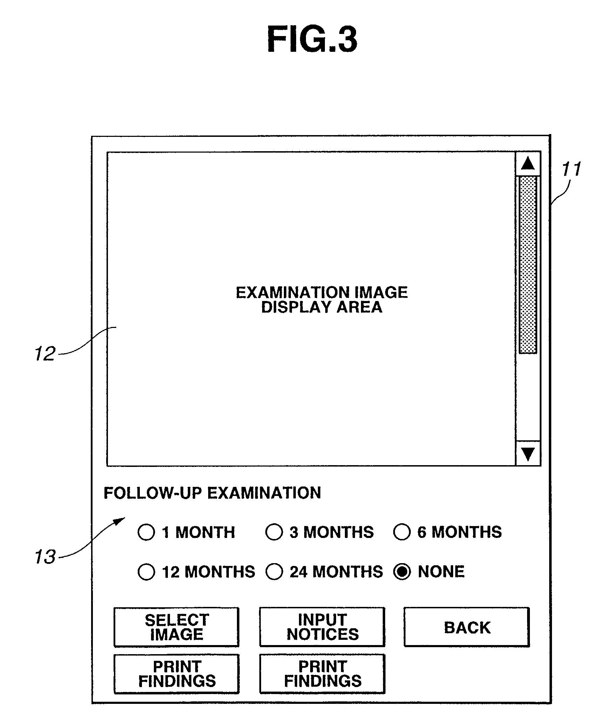

This inputting of whether a follow-up examination is to be given and the time of examination is performed by the doctor operating the filing apparatus 1, and causing a follow-up examination setting window 11 as shown in Figure 3 to be displayed on the monitor of the filing apparatus 1.

The follow-up examination setting window 11 comprises an examination image display area 12 for displaying the patient's endoscopic image based on the patient information, and a follow-up examination setting area 13 for selectively setting whether to have a follow-up examination and the time of examination with radio buttons. The doctor judges the patient's endoscopic image displayed on the examination image display area 12, decides upon the follow-up examination and the time of examination, and selectively sets whether to have a follow-up examination and the time of examination with the radio buttons in the follow-up examination setting area 13 (selects the time of examination: after one month, after three months, after six months, after 12 months, after 24 months, whether to have an examination: NO).

The follow-up examination information, comprising whether to have a follow-up examination and the time of examination which were set, is put into a database form along with the patient information and saved in the filing apparatus 1.

Next, in Step S2, a search of corresponding patients in the prescribed follow-up examination time is initiated with the filing apparatus 1.

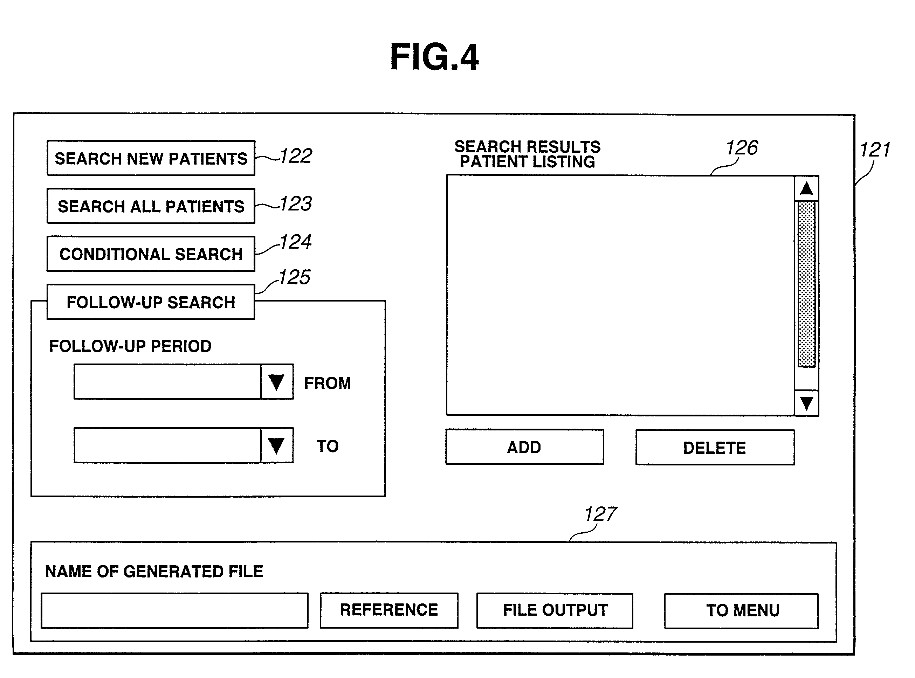

The search causes a follow-up examination search window 121, or follow-up information destination setting window, as shown in Figure 4 to be displayed on the monitor of the filing apparatus 1.

This follow-up examination search window 121 comprises a search new patients button 122, a search all patients button 123, a conditional search button 124, a follow-up period setting button 125, a search results patient listing display area 126, a file generating area 127, and the like.

The search new patients button 122 is a button causing the patients, for whom a file has not been output once, to be displayed on the search results patient listing display area 126.

The search all patients button 123 is a button causing all patients registered in the database to be displayed on the search results patient listing display area 126.

The follow-up period setting button 125 is a button for setting the follow-up examination period, which starts from an input first time and ends on an input second time, to search for patients for whom a follow-up examination is necessary during that period and display the corresponding patients on the search result patient listing display area 126.

The file generating area 127 is a file generating region for creating a file of patient information of patients for whom follow-up is necessary and for outputting the file to the hospital PC 4.

In Step S3, for example, the desired follow-up examination period is set by the filing apparatus 1 using the follow-up period setting button 125, the corresponding patients are searched, and it is determined whether corresponding patients are present. When a corresponding patient is present, in Step S4, a relational data file of the corresponding patient is read with the filing apparatus 1, and the corresponding patient's name and so forth are caused to be displayed on the search results patient listing display area 126. When a corresponding patient is not present, the process ends.

With the process in Step S3, the doctor can search the list of patients for whom a follow-up examination is necessary under various conditions.

Next, in Step S5, the automatic distribution of the follow-up examination guidance and the reservation reception process, discussed below, are executed. In Step S6, when the examination date arrives, the doctor performs the follow-up examination. In Step S7, the doctor judges whether reexamination is necessary and when necessary, the process returns to Step S1. When not necessary, the process ends.

Next, the abovementioned automatic distribution of the follow-up examination guidance and the reservation reception process in Step S5 are explained.

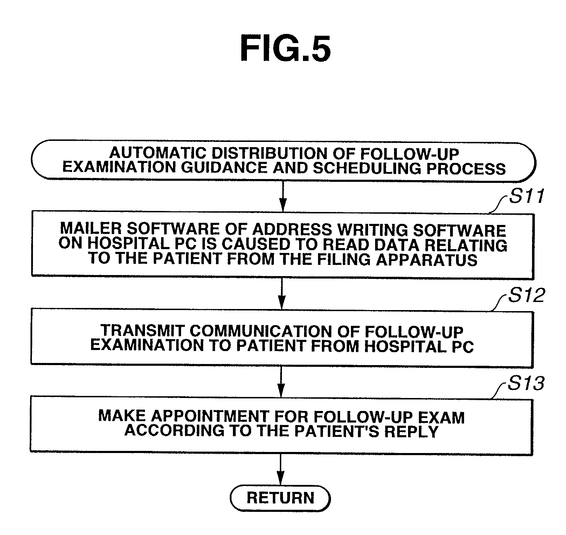

As shown in Figure 5, in the automatic distribution of the follow-up examination guidance and the reservation reception process, the corresponding patient's relational data file read in Step S4 by the filing apparatus 1 is output to the hospital PC 4 in Step S11, and the relational data file is read by the e-mail software or address writing software in the hospital PC 4.

Moreover, the file output of the relational data file to the hospital PC 4 is performed online if there are no problems with security. If there are concerns about security, the file output is performed through a portable storage medium such as a flexible disk.

Next, the follow-up examination guidance is automatically distributed by e-mail or postal mail to the patient's home 6 by the e-mail software or address writing software in the hospital PC 4 in Step S12. In Step S13, the follow-up examination is scheduled based on the follow-up examination guidance according to the response from the patient's home 6 and the process returns to Step S6.

In this way, the present embodiment makes it possible for a doctor to easily search follow-up examinations under various conditions and easily perform planning such as the scheduling of examinations.

Also, with the automatic distribution of a follow-up examination guidance by electronic mail or the like based on the follow-up examination search results for patients for whom follow-up examinations are required, and the reception of reservations from patients, it becomes possible to perform follow-up examinations with certainty, quickly, cheaply and efficiently.

### Second embodiment

A second embodiment is basically the same as the first embodiment and therefore only the different aspects of the constitution are explained. The same symbols are applied to identical aspects of the constitution, and an explanation thereof is omitted.

As shown in Figure 6, the examination follow-up information management apparatus relating to the present invention is constructed in an examination reservation server 23 within an examination scheduling management center 22 connected to a WAN (Wide Area Network) 21 comprising dedicated circuits. The hospital PC 4 is connected to the WAN 21; the WAN 21 is connected to the Internet 5 through a firewall 24. A recording and reproducing apparatus 26 is connected as an examination scheduled patient database to the examination reservation server 23 and files of patient information for whom follow-up examinations are necessary are made into a database in this recording and reproducing apparatus 26. Other elements of the constitution are the same as in the first embodiment. The operation of the present embodiment is explained next. In the present embodiment, the automatic distribution of the follow-up examination guidance and the reservation reception process in Step S5 are different from the first embodiment.

Specifically, the automatic distribution of the follow-up examination guidance and the reservation reception process in the present embodiment, as shown in Figure 7, output the relational data file of the corresponding patient, read from the filing apparatus 1 in Step S4, to the hospital PC 4 in Step S51, and cause the relational data file to be read by the e-mail software on the hospital PC 4.

Moreover, file output of the relational data file to the hospital PC 4 may be performed online if there are no problems with security, and may be performed through a portable storage medium such as a flexible disk if there are concerns about security.

Next, the follow-up examination guidance is automatically distributed (transmitted) one week before the scheduled examination date (ideal follow-up examination date after the follow-up period from the previous examination date) by e-mail to the patient's home 6 from the e-mail software of the hospital PC 4 in Step S52. Meanwhile the patient information of the patient subject to the follow-up examination is transmitted to the examination reservation server 23 by the hospital PC 4.

Accordingly, in the examination reservation server 23, the patient information of the patient subject to a follow-up examination is made into a database and stored in the recording and reproducing apparatus 26, while the examination management data are updated.

Also, the address (URL) of the home page opened to the public by the examination reservation server 23 on the Internet 5 and the access method (ID, password) and so forth are included in the follow-up examination guidance automatically distributed by e-mail.

Next, in Step S53, the patient accesses a home page 31, opened to the public by the examination reservation server 23, with the URL as shown in Figure 8.

Moreover, in the case where the patient does not have a patient's PC (in the case of a patient for which no e-mail address is registered), the same content as in the abovementioned e-mail is distributed by postal mail and the patient accesses the home page 31 by using the PC of an acquaintance or at an Internet cafe or the like.

In Step S54, the patient inputs the ID and password to the home page 31 and clicks on a login button 32. Thereupon, in Step S54, the examination reservation server 23 checks and authenticates the ID and password, and a scheduling calendar page 33 as shown in Figure 9 is opened to the public in Step S55. The patient sets the appointment date by clicking on a date in a scheduling calendar 34.

Moreover, the appointment date can be set only at about two weeks from the previous examination date (the ideal follow-up examination date being after the follow-up period from the previous examination date); the abovementioned ID and password are also only in effect for the period in which the examination can be scheduled. When this period has passed, the examination reservation server 23 invalidates the ID and password which were issued and prevents unauthorized access to the examination reservation server 23. Furthermore, access with this ID and password is limited. The examination reservation server 23 invalidates the ID and password once a predetermined number of access times has been exceeded, even within the allowed period, and prevents unauthorized access to the examination reservation server 23.

Once the appointment date is set in Step S56, a detailed appointment page 35 is opened to the public as shown in Figure 10. The scheduling of the examination is completed with the patient inputting detailed patient information and the appointment time in Step S56, and then the process returns to Step S6.

The doctor (hospital) can inspect the scheduling status of the patient's follow-up examination by accessing the home page 31 on the examination reservation server 23 with the hospital PC 4. Specifically, as shown in Figure 11, the doctor (hospital) accesses the home page 31 shown in Figure 8 and opened to the public by the examination reservation server 23 in Step S81, and inputs the ID and password to the home page 31 in Step S82. The examination reservation server 23 checks and authenticates the ID and password in Step S83. The examination scheduling information page (not shown) having the follow-up examination search window 121 shown in Figure 4 is published in Step S84, and the doctor (hospital) accesses the examination scheduling information page.

Moreover, the hospital ID and password are issued in advance by the examination reservation server 23 in the hospital. The examination reservation server 23 opens to the public the examination scheduling information page, having the follow-up examination search window 121 to the doctor (hospital) based on the input ID and password for the hospital.

The examination scheduling information is searched from the follow-up examination search window 121 of the examination scheduling information page opened to the public in Step S85. The doctor (hospital) confirms and learns the examination scheduling information in Step S86 and terminates the process.

In addition to the effects of the first embodiment, the present embodiment allows for the scheduling of the follow-up examination to be performed on a home page with the examination reservation server 23. This makes it unnecessary for a staff of the hospital 2 to input to a PC the scheduling data from the response by e-mail or postal mail. It becomes possible to confirm appointment information 24 hours a day, 365 days a year, and scheduling can be done more inexpensively, securely, and quickly. Because the examination reservation server 23 manages the examination scheduling information, another effect is that the management burden for the hospital 2 is eliminated. Meanwhile, 24 hour a day, 365 day a year scheduling becomes possible for the patient who wants to make an appointment, and scheduling can be done more inexpensively, securely, and quickly. In the second embodiment, the explanation concerns an endoscopic examination, but the embodiment can also be applied to follow-up examinations for practitioners of other medical care (for example, OO surgery hospital, ρρ neurosurgery clinic, and so forth).

Moreover, as shown in Figure 12, a general practice clinic 41 of a patient, with the patient having been referred to the hospital 2, may be connected to the WAN 21 in the second embodiment and the examination reservation server 23 may issue an ID and password to the clinic 41. The clinic 41 can thereby confirm the endoscopic image and follow-up examination scheduling of a referred patient through the home page using a clinic PC 42. The examination results and examination situation of a referred patient can thereby be learned by the clinic 41.

As shown in Figure 13, the examination reservation server 23 and so forth may also be established in the hospital 2.

Furthermore, the filing apparatus 1 is constituted with a single apparatus, but is not limited to this. As shown in Figure 14, the filing apparatus may also comprise the following in an endoscope examination department 101, for example: an image input and compression apparatus 103 for inputting and compressing images from an endoscope examination apparatus 102; a server 107 for storing images compressed by the image input and compression apparatus 103; a searching/expanding and reproducing apparatus 105 for compressed images stored in the server 107 or in a high volume storage apparatus 104; and a reception PC 106 for receiving examinations with the endoscope examination apparatus 102 from an examination ordering department 116.

At this time, the reception PC 106 is connected to a hospital LAN 111. Likewise, the following are connected to the hospital LAN 111: a PCs of the CT examination department 112, an MRI examination department 113, and so forth, and an internal medicine department 114 for performing medical treatment, which are provided with filing apparatuses; a medical administration and accounting department 115 for performing medical administration and accounting processes; the examination ordering department 116 constituting the examination scheduling management center 22; and a firewall 117 connected to the Internet 5. Moreover, other departments in a hospital besides the internal medicine department may be connected to the hospital LAN 111: otology, dermatology, rehabilitation, and neurosurgery. Examinations by these departments (medical care) may be scheduled by linking the patients and hospital using the examination scheduling management center 22 in the examination ordering department 116. Another possible constitution has an examination scheduling management center 22 established in each department and appointments are made by linking the patients with each department.

### Third embodiment

A third embodiment of the present invention is explained next. The present embodiment comprises the same constitution as the first embodiment.

As shown in Figure 15, the endoscopic image is related to the patient information and stored in the filing apparatus 1 following the examination with the electronic endoscope 3. Thereupon, the doctor determines whether a follow-up examination with the endoscope is necessary for the patient in the filing apparatus 1 in Step S1a. When necessary, the time at which the follow-up examination should be performed and the time for sending the follow-up examination guidance are set. For one follow-up examination, the doctor can set once or a plurality of times of sending the guidance as necessary.

Next, in Step S1b, the destination of the follow-up examination guidance is selected. The setting of whether to have a follow-up examination, the examination time, the guidance sending time, and the guidance destination are displayed on the monitor of the filing apparatus 1 in a follow-up examination setting window 11b as shown in Figure 16.

The follow-up examination setting window 11b comprises: the examination image display area 12 for displaying the endoscopic image of the patient based on the patient information; a follow-up examination setting area 13a for selectively setting with radio buttons whether to have the follow-up examination and the time; and in the case where the time of the follow-up examination is set, a follow-up examination guidance sending time setting area 13b for setting the time for the guidance; a patient information display area 13c for displaying the patient information for the examination; and a referral information display area 13d for setting the referring physician of the patient.

The doctor judges the endoscopic image of the patient displayed in the examination image display area 12, determines whether to have the follow-up examination and the time, and sets whether to have the follow-up examination and the time selectively with radio buttons in the follow-up examination setting area 13a.

Next, in the follow-up examination guidance sending time setting area 13b, the sending time of the guidance, one or a plurality of times, is set. For example, a follow-up examination is set for six months later, the follow-up examination guidance can be set to be sent twice, one month and one week before.

Furthermore, the referring physician of the patient is set in the referral information display area 13d for the sending of the follow-up examination guidance. Information such as the name of the referring physician, as well as the e-mail addresses and postal address, can be set as the referring physician information.

Destinations for the follow-up examination guidance can be set to include a plurality of CCs (carbon copies) by pressing a CC setting button 13e. Like for the referring physician, information such as the name, e-mail address, and postal address can be set for the CC destinations.

The follow-up examination information, comprising the settings for whether to have the follow-up examination, the time, the guidance sending time, and the referral information, is made into a database along with the patient information and saved in the filing apparatus 1.

Operations following Step S2 are the same as operations following Step S2 in the first embodiment, except that the follow-up examination period is expressed as the time for sending the follow-up examination guidance.

When sending the follow-up examination guidance in Step S12, printed guidance may also be mailed out. In this case, guidance is automatically printed in a quantity appropriate for the patient, referring physician, and people receiving a carbon copy set in Step S1b.

In addition to the effects of the first embodiment, the present embodiment can ensure the notification of the patient regarding the follow-up examination even in the case where the follow-up examination is scheduled to be performed after a long period of time.

In the case where the patient undergoes the examination at another hospital on the referral of a general practitioner, it may sometimes be the case that the general practitioner needs to learn of the referred patient's situation thereafter. In that case as well, the general practitioner can learn of the patient's situation including the follow-up examination.

Having described the preferred embodiments of the invention referring to the accompanying drawings, it should be understood that the present invention is not limited to those precise embodiments and various changes and modifications thereof could be made by one skilled in the art without departing from the spirit or scope of the invention as defined in the appended claims.

## Claims

1. An examination follow-up information management apparatus, for managing follow-up examinations of a subject, **characterized by** comprising:
a display for displaying examination images of said subject;
a follow-up information input window display unit for displaying a follow-up information input window, for inputting follow-up information for the examination for said subject, on said display;
a follow-up information input unit for inputting follow-up information for said subject in said follow-up information input window displayed on said follow-up information input window display unit; and
a follow-up information memory for storing said follow-up information input with said follow-up information input unit as follow-up information for said subject.

2. The examination follow-up information management apparatus according to Claim 1, further **characterized by** comprising a follow-up information communicating unit for communicating said follow-up information stored in said follow-up information memory through a communication network.

3. The examination follow-up information management apparatus according to Claim 2, further **characterized by** comprising:
an examination reservation password information communicating unit for adding examination scheduling password information to said follow-up information transmitted by said follow-up information communicating unit and sending this information through said communication network; and
examination reservation unit for executing examination reservation based on said reservation password information transmitted through said communication network.

4. The examination follow-up information management apparatus according to Claim 1, further **characterized by** comprising:
a follow-up information destination setting window display for displaying a follow-up information destination setting window, for setting a destination of said follow-up information, on said display;
a follow-up information destination setting unit for setting a destination information of said follow-up information to said follow-up information destination setting window displayed in said follow-up information destination setting window display unit;
destination information memory for storing said destination information set by said follow-up information destination setting unit as the follow-up information destination information for said subject; and
a follow-up information communicating unit for transmitting said follow-up information stored in said follow-up information memory through a communication network according to said destination information stored in said destination memory.

5. The examination follow-up information management apparatus according to Claim 4:
wherein said follow-up information destination setting unit is able to set a plurality of destination information including said subject;
and further comprising:
a destination information memory for storing said destination information, set by said follow-up information destination setting unit as the follow-up information destination for said subject; and
a follow-up information communicating unit for transmitting said follow-up information stored in said follow-up information memory through the communication network gradually according to said plurality of destination information stored in said destination memory.

6. The examination follow-up information management apparatus according to Claim 1, further **characterized by** comprising:
a follow-up searching unit for searching said subjects for whom follow-up is necessary based on said follow-up information stored in said follow-up information memory.

7. The examination follow-up information management apparatus according to Claim 2, further **characterized by** comprising:
a follow-up searching unit for searching said subjects for whom follow-up is necessary based on said follow-up information stored in said follow-up information memory; and
wherein said follow-up information communicating unit communicates through the communication network said follow-up information stored in said follow-up information memory to said subject for whom follow-up is necessary, and that was searched by said follow-up searching unit.

8. The examination follow-up information management apparatus according to Claim 4, further **characterized by** comprising:
a follow-up searching unit for searching for said subjects for whom follow-up is necessary based on said follow-up information stored in said follow-up information memory; and
wherein through the communication network, and according to said destination information stored in said destination memory, said follow-up information communicating unit transmits said follow-up information stored in said follow-up information memory with respect to said subject for whom follow-up is necessary, and that was searched by said follow-up searching unit.

9. A control method for an examination follow-up information management apparatus, for managing follow-up examinations of a subject, **characterized by** comprising:
an examination image display process for displaying the examination image of said subject on a display;
a follow-up information input window display process for displaying a follow-up information input window, for inputting the examination follow-up information for said subject, on said display;
a follow-up information input process for inputting follow-up information for said subject to said follow-up information input window displayed by said follow-up information input window display process; and
a follow-up information storing process for storing said follow-up information input with said follow-up information input process as follow-up information for said subject.

10. The examination follow-up information management method, according to Claim 9, further **characterized by** comprising a follow-up information communicating process for communicating said follow-up information, stored in said follow-up information storing process, through a communication network.

11. The examination follow-up information management method, according to Claim 10, further **characterized by** comprising:
an examination reservation password information communicating process for adding examination reservation password information to said follow-up information, sent by said follow-up information communicating process, and sending the password information through said communication network; and
an examination reservation process for scheduling the examination based on said scheduling password information sent through said communication network.

12. A control method for the examination follow-up information management apparatus according to Claim 9, further **characterized by** comprising:
a follow-up information destination setting window display process for displaying the follow-up information destination setting window, for setting a destination for said follow-up information, on said display;
a follow-up information destination setting process for setting a destination information of said follow-up examination to said follow-up information destination setting window displayed in said follow-up information destination setting window display process;
a destination information storing process for storing said destination information, set in said follow-up information destination setting process, as the destination information of the follow-up information for said subject; and
a follow-up information communicating process for communicating said follow-up information stored in said follow-up information storing process through a communication network according to said destination information stored in said address storing process.

13. The control method for the examination follow-up information management apparatus, according to Claim 12:
wherein a plurality of destination information including that for said subject can be set in said follow-up information destination setting process;
the control method further **characterized by** comprising:
a destination information storing process for storing said destination information, set in said follow-up information destination setting process, as the destination information of the follow-up information for said subject; and
a follow-up information communicating process for transmitting said follow-up information stored in said follow-up information storing process through a communication network gradually according to said plurality of destination information stored in said destination information storing process.

14. The control method for the examination follow-up information management apparatus, according to Claim 9, further **characterized by** comprising a follow-up search process for searching said subjects for whom follow-up is necessary based on said follow-up information stored in said follow-up information storing process.

15. The control method for the examination follow-up information management apparatus, according to Claim 10, further **characterized by** comprising a follow-up search process for searching said subjects for whom follow-up is necessary based on said follow-up information stored in said follow-up information storing process; and
wherein said follow-up information communicating process communicates said follow-up information, stored in said follow-up information storing process, through a communication network with respect to said subject for whom follow-up is necessary and who was searched in said follow-up search process.

16. The control method for the examination follow-up information management apparatus, according to Claim 12, further **characterized by** comprising a follow-up search process for searching said subjects for whom follow-up is necessary based on said follow-up information stored in said follow-up information storing process; and
wherein said follow-up information communicating process communicates said follow-up information, stored in said follow-up information storing process, through a communication network with respect to said subject for whom follow-up is necessary and who was searched in said follow-up search process, according to said destination information stored in said destination information storing process.
